# EUROPEAN PATENT APPLICATION

(11) **EP 4 234 681 A1**
(43) Date of publication of application: **30.08.2023**
(21) Application number: 21898573.7
(22) Date of filing: 24.11.2021
(51) Int. Cl.: C12N 5/00, C12N 5/071, C12M 3/00, C12M 1/12

(54) **EX-VIVO LIVER DISEASE MODEL USING TRIPLE CO-CULTURE AND CONSTRUCTION METHOD THEREFOR**

(30) Priority: 26.11.2020 KR 20200161490
(71) Applicant: Future Medicine Co., Ltd., Seongnam-si, Gyeonggi-do 13449 (KR)
(72) Inventor: CHOI, Yoon, Pyo, Cheonan-si Chungcheongnam-do 31152 (KR); SEO, Seong, Wook, Gwangju-si Gyeonggi-do 12773 (KR); AHN, Sang, Yeop, Seongnam-si Gyeonggi-do 13449 (KR); PARK, Da, In, Suwon-si Gyeonggi-do 16517 (KR); LEE, Myeong, Hoon, Suwon-si Gyeonggi-do 16508 (KR)
(74) Representative: Roos, Peter
(86) International application number: PCT/KR2021/017325
(87) International publication number: WO 2022/114747

(57) **Abstract**

The present invention relates to an *in vitro* liver disease model using triple co-culture and a preparation method thereof, and the method of preparing the *in vitro* liver disease model includes directly co-culturing hepatocytes and hepatic stellate cells, and simultaneously indirectly co-culturing Kupffer cells by being separated from the hepatocytes and the hepatic stellate cells to enable a rapid and highly accurate analysis by similarly implementing the *in vivo* environment compared to mono-culture, and simultaneously make it easier to collect and measure specimens than typical direct co-culture.

## Description

### BACKGROUND

### 1. Field of the Invention

The present invention relates to an *in vitro* liver disease model using triple co-culture and a preparation method thereof, and more specifically, to an *in vitro* liver disease model using triple co-culture, which enables a rapid and highly accurate analysis by similarly implementing the *in vivo* environment compared to mono-culture, and simultaneously makes it easier to collect and measure specimens than typical direct co-culture, and a preparation method thereof.

### 2. Discussion of Related Art

A model in which liver disease is induced is typically used in preclinical experiments performed to analyze and evaluate the effects of specific drugs on the liver. However, it is difficult to reproduce the complexity of liver tissue, and in particular, it is even more difficult to implement a non-alcoholic steatohepatitis (NASH) or non-alcoholic fatty liver disease (NAFLD) model, in which symptoms such as inflammation, fibrosis, and steatosis act in a complex manner.

Various hepatocyte culture methods have been studied for this purpose, and examples thereof include 2D mono-culture in which only one type of cell is simply cultured in a culture dish, 2D co-culture in which various types of cells are mixed and cultured in a culture dish, 3D mono-culture in which one type of cell is cultured by constituting a 3D structure, 3D co-culture in which various types of cells are cultured by constituting a 3D structure, and the like.

3D co-culture has drawn attention for its potential to mimic the *in vivo* environment and experimental results more similarly than 2D mono-culture. However, the complexity increases from 2D mono-culture to 3D co-culture, whereas it is difficult to verify the state, expression pattern, and the like of each cell individually, so that there is a disadvantage in that the cells are not easily analyzed and the cost is high.

Since it has been proven that when the expression rate of A₃AR in the liver is confirmed, A₃AR shows a predominant expression rate in Kupffer cells and fibrosis-inducing hepatic stellate cells in liver tissue and the overexpression of A₃AR is closely associated with liver inflammation and fibrosis, the suppression of A₃AR ligand overexpression is highly likely to produce a therapeutic agent for inflammation and fibrosis.

As non-alcoholic steatohepatitis (NASH) is aggravated, NASH progresses to hepatocellular carcinoma (HCC), but it was confirmed that the expression rate of A₃AR in peripheral blood mononuclear cells (PBMCs) of human hepatocellular carcinoma cancer (HCC) patients was significantly increased compared to normal subjects (Int J Oncol. 2008 Aug;33(2):287-95.).

Since the overexpression of A₃AR in NASH patients is highly correlated with a disease, the suppression of A₃AR overexpression is closely associated with the treatment of the disease.

### SUMMARY OF THE INVENTION

Accordingly, a problem to be solved by the present invention is to provide an *in vitro* liver disease model using triple co-culture, which enables a rapid and highly accurate analysis by similarly implementing the *in vivo* environment compared to mono-culture, and simultaneously makes it easier to collect and measure specimens than typical direct co-culture, and a preparation method thereof.

The problems of the present invention are not limited to the aforementioned technical problems, and other technical problems, which have not been mentioned, may be clearly understood by a person with ordinary skill in the art from the following description.

A method of preparing an *in vitro* liver disease model using triple co-culture according to an embodiment of the present invention to solve the above problem includes directly co-culturing hepatocytes and hepatic stellate cells, and simultaneously indirectly co-culturing Kupffer cells by being separated from the hepatocytes and the hepatic stellate cells.

The hepatocytes and the hepatic stellate cells are cultured so as to contact each other, and the Kupffer cells are cultured so as not to contact the hepatocytes and the hepatic stellate cells, provided that the hepatocytes, the hepatic stellate cells, and the Kupffer cells may share the same medium.

The hepatocytes may be one or more of HepG2 cells and HuH-7 cells.

The hepatic stellate cells may be LX-2 cells.

The Kupffer cells may be immortalized Kupffer cells.

The method may further include inducing steatosis, inflammation or fibrosis in the hepatocytes, the hepatic stellate cells, and/or the Kupffer cells.

The inducing of the steatosis may be treating the hepatocytes, the hepatic stellate cells, and/or the Kupffer cells with a free fatty acid (FFA), the inducing of the inflammation may be treating the hepatocytes, the hepatic stellate cells, and/or the Kupffer cells with a FFA and a lipopolysaccharide (LPS), and the inducing of the fibrosis may be treating the hepatocytes, the hepatic stellate cells, and/or the Kupffer cells with a FFA and TGF-β1.

The method may further include treating the hepatocytes, the hepatic stellate cells, and/or the Kupffer cells with one or more agonists, partial agonists, antagonists, or partial antagonists selected from the group consisting of A₁AR, A_{2A}AR, A_{2B}AR, A₃AR, β-arrestin, FXR and THR-β.

The method may further include measuring one or more markers selected from the group consisting of steatosis, inflammation and fibrosis for the hepatocytes, the hepatic stellate cells and/or the Kupffer cells.

The marker may be one or more selected from the group consisting of A₃AR, FXR, THR-β, CCL2, LOX, COL1A1, COL4A1, IL-6, IL-1β, TNF-α, ACTA2, TIMP1, TΠVVIP2, TGF-β1, CXCL8, lipid, collagen, reactive oxygen species (ROS), α-SMA and fibronectin.

After 144 hours from the start of culture, the cell number ratio of the hepatocytes to the hepatic stellate cells may be 2 to 7 : 1.

After 144 hours from the start of culture, the cell number ratio of the sum of the hepatocytes and the hepatic stellate cells to the Kupffer cells may be 1 : 1 to 5.

The liver disease may be non-alcoholic steatohepatitis (NASH) and/or non-alcoholic fatty liver disease (NAFLD).

An *in vitro* liver disease model using triple co-culture according to an embodiment of the present invention to solve the other problems includes: a first incubator containing hepatocytes and hepatic stellate cells; and a second incubator containing Kupffer cells, which are disposed while being separated from the hepatocytes and the hepatic stellate cells, in which the *in vitro* liver disease model has pores with a size smaller than the Kupffer cell, which are formed on the bottom surface of the second incubator, and further includes a medium which fills both the first incubator and the second incubator through the pores.

The pores may have an average size of 0.1 to 0.6 µm.

The medium may include one or more selected from the group consisting of fetal bovine serum (FBS), penicillin/streptomycin (P/S), glutamine, glucose, sodium pyruvate, P/S (HyClone^{™}), Dulbecco's Modified Eagle's Medium, EmbryoMax^{™} 2 mM L-glutamine, EmbryoMax^{™} ES cell qualified FBS and EmbryoMax^{™} DMEM high glucose.

Specific details of other embodiments are included in the detailed description.

### BRIEF DESCRIPTION OF THE DRAWINGS

The above and other objects, features and advantages of the present invention will become more apparent to those of ordinary skill in the art by describing in detail exemplary embodiments thereof with reference to the accompanying drawings, in which:
FIG. 1 is a schematic view illustrating a method of preparing an *in vitro* liver disease model using triple co-culture according to an embodiment of the present invention;
FIG. 2 shows a schematic view of direct co-culture of a human hepatocellular carcinoma cell line and a human hepatic stellate cell line according to experimental examples of the present invention and images of cells for each checkpoint;
FIG. 3 is a schematic view illustrating the difference between mono-culture and triple co-culture methods according to experimental examples of the present invention;
FIG. 4 is a schematic view illustrating cell culture, a free fatty acid (FFA) treatment procedure, and measurement checkpoints according to experimental examples of the present invention;
FIGS. 5 to 7 are graphs measuring the expression levels of A₃AR, FXR, THR-β and lipid in cells in which hepatic steatosis was induced according to experimental examples of the present invention;
FIGS. 8 and 9 are graphs measuring the expression levels of CCL2, IL-1β, IL-6, A₃AR, and the like in cells in which hepatic inflammation was induced according to experimental examples of the present invention; and
FIGS. 10 to 14 are graphs measuring the expression levels of CCL2, IL-1β, IL-6, A₃AR, collagen, and the like in cells in which hepatic fibrosis was induced according to experimental examples of the present invention.

### DETAILED DESCRIPTION OF EXEMPLARY EMBODIMENTS

The benefits and features of the present invention, and the methods of achieving the benefits and features will become apparent with reference to embodiments to be described below in detail. However, the present invention is not limited to the embodiments to be disclosed below and may be implemented in various other forms, and the embodiments are only provided for rendering the disclosure of the present invention complete and for fully representing the scope of the invention to a person with ordinary skill in the technical field to which the present invention pertains, and the present invention will be defined only by the scope of the claims.

The terms used in the present specification are used merely to describe embodiments, and are not intended to limit the present invention. In the present specification, the 'and/or' includes each and all combinations of one or more of the items mentioned. Further, the singular form also includes the plural forms unless specifically stated in a phrase. The terms 'comprises' and/or 'comprising' used in the specification do not exclude the presence or addition of one or more other constituent elements in addition to the referenced constituent elements. The numerical range indicated by using '-' or 'to' indicates a numerical range including values described before and after it as a lower limit and an upper limit, respectively, unless otherwise stated. 'About' or 'approximately' means a value or numerical range within 20% of the value or numerical range described thereafter.

Further, in describing the constituent elements of the examples of the present invention, terms such as first, second, A, B, (a), and (b) may be used. These terms are merely for distinguishing one constituent element from another, and the nature, turn, or order of the corresponding constituent element is not limited by the term.

Unless otherwise defined, all the terms (including technical and scientific terms) used in the present specification will be able to be used as a meaning which may be commonly understood to a person with ordinary skill in the art to which the present invention pertains. In addition, the terms defined in generally used dictionaries are not to be interpreted ideally or excessively unless clearly and specifically defined.

Moreover, in describing the examples of the present invention, when it is determined that the specific description of relevant known configurations or functions obstructs the understanding for the examples of the present invention, the detailed description thereof will be omitted.

As used herein, the term 'mono-culture' may mean that a single type of cell is cultured in an incubator or the like and does not interact with other cells.

As used herein, the term 'co-culture' may mean that interactions between heterogeneous cells are induced by mixing and culturing two or more types of cells in an incubator.

As used herein, the term 'direct co-culture' may refer to co-culture that enables interactions between heterogeneous cells using secreted materials such as cytokines, and enables substantial physical contact.

As used herein, the term `indirect co-culture' may refer to co-culture that enables interactions between heterogeneous cells using secreted materials such as cytokines by sharing the same medium, and the like, but does not bring the heterogeneous cells into contact with each other because they are physically separated.

As used herein, the term 'control' may refer to, for example, a mono-culture model, in which different conditions are the same as those of the culture method of the present invention, but the heterogeneous cells are only differently disposed, and the like.

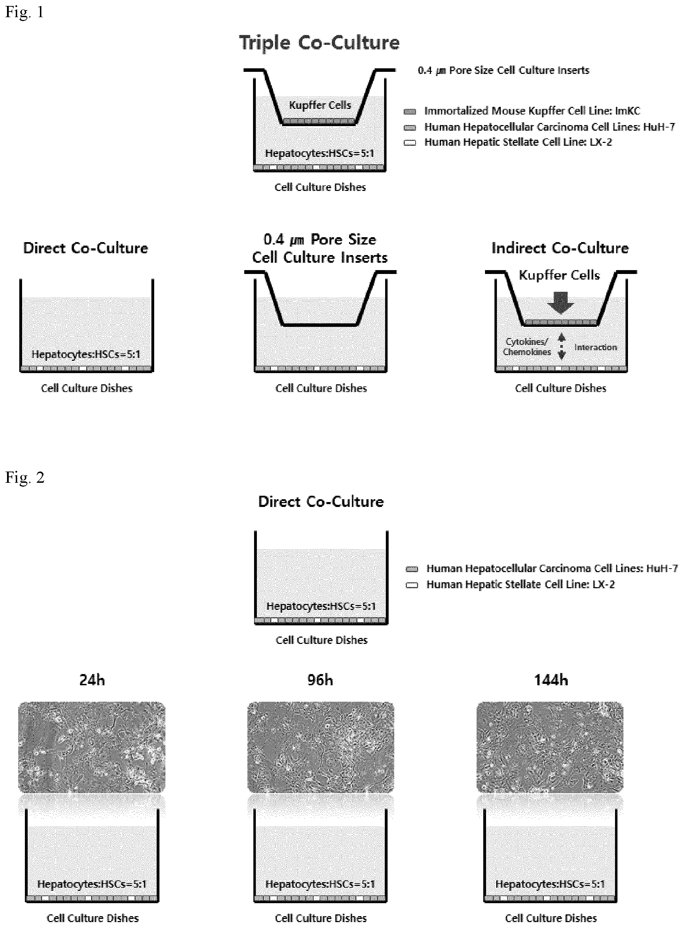

FIG. 1 is a schematic view illustrating a method of preparing an *in vitro* liver disease model using triple co-culture according to an embodiment of the present invention.

Referring to FIG. 1, an *in vitro* liver disease model using the triple co-culture of the present invention may be implemented by directly co-culturing hepatocytes and hepatic stellate cells, and simultaneously indirectly co-culturing Kupffer cells by being separated from the hepatocytes and the hepatic stellate cells.

The model may be implemented through a first incubator containing hepatocytes and hepatic stellate cells and a second incubator containing Kupffer cells. The second incubator is disposed while being separated from the hepatocytes and the hepatic stellate cells, and has pores with a size smaller than the Kupffer cell, which are formed on the bottom surface thereof, and, and through the pores, both the first incubator and the second incubator may be filled with a medium. In exemplary embodiments, the second incubator may be disposed at the top in the first incubator while being separated from the hepatocytes and the hepatic stellate cells, and the medium may be filled to a height which allows the Kupffer cells in the second incubator to be submerged.

The medium may include one or more selected from the group consisting of fetal bovine serum (FBS), penicillin/streptomycin (P/S), glutamine, glucose, sodium pyruvate, P/S (HyClone^{™}), Dulbecco's Modified Eagle's Medium, EmbryoMax^{™} 2 mM L-glutamine, EmbryoMax^{™} ES cell qualified FBS and EmbryoMax^{™} DMEM high glucose. In exemplary embodiments, each cell may be cultured in a medium including FBS in the separately culturing of each cells before the triple co-culture step, and a medium including one or more selected from the group consisting of P/S (HyClone^{™}) and Dulbecco's Modified Eagle's Medium may be used in the triple co-culture step. In this case, the concentration of FBS may be gradually reduced to 1% before the triple co-culture step. However, the embodiments of the present invention are not limited thereto.

Accordingly, hepatocytes and hepatic stellate cells can be mixed and directly interacted with each other for direct co-culture, and indirect co-culture can be performed between hepatocytes/hepatic stellate cells and Kupffer cells, in which they interact with each other using secreted materials such as cytokines.

The secreted materials such as cytokines may move through the pores. The average size of the pores may be 0.6 µm or less, preferably 0.1 to 0.6 µm, and more preferably about 0.4 µm, but is not limited thereto, and pores may also be formed in other sizes that allow secreted materials such as cytokines to pass while physically separating Kupffer cells from hepatocytes and hepatic stellate cells.

The hepatocytes may be one or more of HepG2 cells and HuH-7 cells (human hepatocellular carcinoma cells), and the hepatic stellate cells may be LX-2 cells (human hepatic stellate dells).

The hepatocytes, the hepatic stellate cells and the Kupffer cells may each be independently human or animal-derived cells (primary cells) or immortalized cells.

In some embodiments, the Kupffer cells may be immortalized Kupffer cells.

The cell number ratio of hepatocytes to hepatic stellate cells may be 2 to 7 : 1, specifically 3 to 6 : 1, and the cell number ratio of the sum of hepatocytes and hepatic stellate cells to Kupffer cells may be 1 : 0.5 to 5, specifically 1 : 1 to 5.

The hepatocytes, the hepatic stellate cells and the Kupffer cells may be cultured at 35 to 39°C, preferably about 37°C, and may be cultured under the conditions of 2 to 8% CO₂, preferably about 5% CO₂. Since the optimum culture conditions are different for each cell type, it requires very stringent conditions to culture the three types of cells together, and when the above conditions are not satisfied, at least some cell types may not be properly cultured.

The liver disease model of the present invention may be prepared for screening materials having preventive or therapeutic efficacy against liver diseases such as non-alcoholic steatohepatitis (NASH) and/or non-alcoholic fatty liver disease (NAFLD). In particular, the liver disease model using the triple co-culture of the present invention may more similarly implement the *in vivo* environment by reflecting interactions among hepatocytes, hepatic stellate cells and Kupffer cells. Accordingly, when the cells are treated with a free fatty acid (FFA) or the like to induce non-alcoholic steatohepatitis (NASH) and/or non-alcoholic fatty liver disease (NAFLD), related markers may be rapidly and accurately expressed, and high responsiveness and more concentration dependent response may be exhibited. Similarly, even when a model in which non-alcoholic steatohepatitis (NASH) and/or non-alcoholic fatty liver disease (NAFLD) are/is induced is treated with an active material to alleviate non-alcoholic steatohepatitis (NASH) and/or non-alcoholic fatty liver disease (NAFLD), related markers may be rapidly and accurately expressed. Therefore, when the model of the present invention is used, a reliable and time-saving analysis can be performed. That is, the present invention includes a method of analyzing an *in vitro* liver disease model prepared using triple co-culture.

Non-alcoholic steatohepatitis (NASH) and/or non-alcoholic fatty liver disease (NAFLD) are/is a disease in which symptoms such as inflammation, fibrosis and steatosis act in a complex manner, and the efficacy, pharmacological mechanism, and the like of a specific drug may be more accurately determined only when the related markers of the symptoms should be comprehensively considered. The liver disease model of the present invention can more rapidly and accurately express inflammation, fibrosis and steatosis-related markers, thereby enabling NASH and/or NAFLD to be highly reliably and efficiently analyzed.

In an embodiment, when steatosis, inflammation, and the like are induced by treating the cells with FFA, and the like, the liver disease model of the present invention may improve the expression level and/or expression rate of A₃AR, FXR, THR-β, CCL2, LOX, COL1A1, COL4A1, IL-6, IL-1β, TNF-α, ACTA2, TIMP1, lipid, collagen, and the like, which are important markers for the analysis of non-alcoholic steatohepatitis (NASH) and/or non-alcoholic fatty liver disease (NAFLD), compared to the control.

In addition, when cells are treated with a specific drug (for example, A₃AR ligand) whose mechanism of action is verified through *in vivo* experiments, changes in the expression level and/or expression rate of related markers may be greater than in the control, and may exhibit a more concentration-dependent tendency. That is, there is an advantage of being able to show results closer to the *in vivo* environment than the control.

In some embodiments, the liver disease model of the present invention may improve the secretion amount or secretion rate of TNF-α, IL-6, IL-1β, and the like, which are pro-inflammatory cytokines important for the analysis of inflammation, compared to the control. In specific embodiments, the inflammatory markers may be analyzed by measuring changes in reactive oxygen species (ROS) using DCFDA cellular ROS detection assay, measuring changes in mRNA through qRT-PCR, measuring changes in protein through enzyme-linked immunosorbent assay (ELISA), and the like.

Independently of this, the liver disease model of the present invention may improve the secretion amount or secretion rate of TGF-β1, α-SMA, Collagen Type-I, TIMP-2, fibronectin, and the like, which are pro-inflammatory cytokines important for the analysis of fibrosis, compared to the control. In specific embodiments, the fibrosis markers may be analyzed by measuring changes in mRNA through qRT-PCR, measuring changes in protein through ELISA, and the like.

In addition, fat accumulation by FFA treatment may significantly and rapidly change one or more of intracellular lipid content, intracellular collagen, and intracellular ROS, compared to the control. In specific embodiments, steatosis may be analyzed through Oil Red O staining.

In some embodiments, the markers may be measured 144 hours after FFA treatment.

The liver disease model of the present invention has an advantage in that specimens related to steatosis, inflammation, fibrosis, and the like are easily collected and measured by culturing Kupffer cells physically separated from hepatocytes and hepatic stellate cells by an indirect co-culture method.

That is, the triple co-culture of the present invention implements the *in vivo* environment more similarly than mono-culture and enables rapid analysis, and simultaneously, collects and measures specimens more easily than typical direct co-culture.

Hereinafter, embodiments of the present invention will be described in detail with reference to Preparation Examples and Experimental Examples, but it is obvious that the effects of the present invention are not limited by the following Experimental Examples.

### Experimental Example 1: Cell culture and Subculture

### Experimental Example 1-1: Cell culture and subculture of HuH-7 human hepatocellular carcinoma cell line

### <HuH-7 human hepatocellular carcinoma cell line: Cell culture>

The HuH-7 human hepatocellular carcinoma cell line (JCRB Cell Bank, Japan) used in the experiment was cultured in a CO₂ incubator (Thermo Scientific^{™}) under 37°C and 5% CO₂ conditions using Dulbecco's Modified Eagle's Medium [+] 4.5 g/L glucose, L-glutamine and sodium pyruvate (DMEM, Corning) containing 1% P/S (HyClone^{™}) and 10% fetal bovine serum (FBS, Gibco).

### <HuH-7 human hepatocellular carcinoma cell line: Subculture>

A cell culture dish was taken out from the CO₂ incubator (Thermo Scientific^{™}) and washed once with 1X DPBS (HyClone^{™}) after discarding all the cell culture medium. All DPBS was discarded, 1X trypsin-EDTA protease solution (HyClone^{™}) was added thereto, and the dish was left to stand in a CO₂ incubator (Thermo Scientific^{™}) under 37°C and 5% CO₂ conditions for 3 minutes. A new complete medium and cells were mixed in a conical tube (SPL), and the resulting mixture was centrifuged at 1300 rpm for 3 minutes. After all the supernatant was removed, a new complete medium was added thereto, the cells were resuspended by tapping or pipetting, and cell viability and cell number were measured using an automated cell counter (Logos Biosystems). Thereafter, the cells and a culture solution were added to a new culture dish such that the number of cells per area was 2.5 x 10⁴ cells/cm², the culture dish was well shaken, and then the resulting mixture was cultured in a CO₂ incubator (Thermo Scientific^{™}) under 37°C and 5% CO₂ conditions. The subculture of the HuH-7 cell line was limited to no more than 10 passages.

### Experimental Example 1-2: Cell culture method and subculture of ImKC immortalized mouse Kupffer cell line

### <ImKC immortalized mouse Kupffer cell line: Cell culture>

The ImKC immortalized mouse Kupffer cell line (Sigma-Aldrich) used in the experiment was cultured in a CO₂ incubator (Thermo Scientific^{™}) under 37°C and 5% CO₂ conditions using Dulbecco's Modified Eagle's Medium [+] 4.5 g/L glucose, L-glutamine and sodium pyruvate (DMEM, Corning) containing 1% P/S (HyClone^{™}) and 10% fetal bovine serum (FBS, Gibco).

### <ImKC immortalized mouse Kupffer cell line: Subculture>

A cell culture dish was taken out from the CO₂ incubator (Thermo Scientific^{™}) and washed once with 1X DPBS (HyClone^{™}) after discarding all the cell culture medium. All DPBS was discarded, 1X trypsin-EDTA protease solution (HyClone^{™}) was added thereto, and the dish was left to stand in a CO₂ incubator (Thermo Scientific^{™}) under 37°C and 5% CO₂ conditions for 3 minutes. A new complete medium and cells were mixed in a conical tube (SPL), and the resulting mixture was centrifuged at 1300 rpm for 3 minutes. After all the supernatant was removed, a new complete medium was added thereto, the cells were resuspended by tapping or pipetting, and cell viability and cell number were measured using an automated cell counter (Logos Biosystems). Thereafter, the cells and a culture solution were added to a new culture dish such that the ratio was 1/6 to 1/10 of the original number of cells, the culture dish was well shaken, and then the resulting mixture was cultured in a CO₂ incubator (Thermo Scientific^{™}) under 37°C and 5% CO₂ conditions. The subculture of the ImKC was limited to no more than 10 passages.

### Experimental Example 1-3: Cell culture and subculture of LX-2 human hepatic stellate cell line

### <LX-2 human hepatic stellate cell line: Cell culture>

The LX-2 human hepatic stellate cell line (Sigma-Aldrich) used in the experiment was cultured in a CO₂ incubator (Thermo Scientific^{™}) under 37°C and 5% CO₂ conditions using Dulbecco's Modified Eagle's Medium [+] 4.5 g/L glucose, L-glutamine and sodium pyruvate (DMEM, Corning) containing 1% P/S (HyClone^{™}) and 2% fetal bovine serum (FBS, Gibco).

### <LX-2 human hepatic stellate cell line: Subculture>

A cell culture dish was taken out from the CO₂ incubator (Thermo Scientific^{™}) and washed once with 1X DPBS (HyClone^{™}) after discarding all the cell culture medium. All the DPBS was discarded, an accutase solution (DPBS (0.2 g/L KCl, 0.2 g/L KH₂PO₄, 8 g/L NaCl, and 1.15 g/L Na₂HPO₄) containing 0.5 mM EDTA 4Na and 3 mg/L Phenol Red, Sigma-Aldrich) was added thereto, and the cell culture dish was left to stand in a CO₂ incubator (Thermo Scientific^{™}) under 37°C and 5% CO₂ conditions for 3 minutes. A new complete medium and cells were mixed in a conical tube (SPL), and the resulting mixture was centrifuged at 1300 rpm for 3 minutes. After all the supernatant was removed, a new complete medium was added thereto, the cells were resuspended by tapping or pipetting, and cell viability and cell number were measured using an automated cell counter (Logos Biosystems). Thereafter, the cells and a culture solution were added to a new culture dish such that the ratio was 1/3 to 1/6 of the original number of cells, the culture dish was well shaken, and then the resulting mixture was cultured in a CO₂ incubator (Thermo Scientific^{™}) under 37°C and 5% CO₂ conditions. The subculture of the LX-2 cell line was limited to no more than 10 passages.

### Experimental Example 2: Cellular in vitro NASH model: Triple co-culture system

### Experimental Example 2-1: Preparation of free fatty acid (FFA; oleic/palmitic acid)-conjugated bovine serum albumin (BSA) stock solution

### <Preparation of 6.6 mM oleic acid BSA-conjugated stock solution>

317.5 µL of oleic acid (C18:1) (MW 282.46, Sigma-Aldrich, 3.15 M) was mixed with 10 ml of 0.1 N NaOH (Sigma-Aldrich), and then the resulting mixture was dissolved in a water bath at 70°C to prepare a 100 mM oleic acid solution. 200 µL of the 100 mM oleic acid solution was slowly added to a 10% fatty acid-free bovine serum albumin (Sigma-Aldrich) solution in a water bath at 55°C, and conjugated to BSA. The pH of the solution was adjusted to 7.4 using 1 NaOH (Sigma-Aldrich). 1 ml of the solution was each aliquoted and stored in a deep freezer ultra-low freezer (TSX Series, Thermo Scientific^{™}).

### <Preparation of 3.3 mM palmitic acid BSA-conjugated stock solution>

128.2 mg of palmitic acid (C16:0) (MW 256.42, Sigma-Aldrich) was mixed with 10 ml of 0.1 N NaOH (Sigma-Aldrich), and then the resulting mixture was dissolved in a water bath at 70°C to prepare a 50 mM palmitic acid solution. 200 µL of the 50 mM palmitic acid solution was each slowly added to a 10% fatty acid-free bovine serum albumin (Sigma-Aldrich) solution in a water bath at 55°C, and conjugated to BSA. The pH of the solution was adjusted to 7.4 using 1 NaOH (Sigma-Aldrich). 1 ml of the solution was each aliquoted and stored in a deep freezer ultra-low freezer (TSX Series, Thermo Scientific^{™}).

### Experimental Example 2-2: Preparation of lipopolysaccharide (LPS) stock solution

Distilled water was added to LPSO111:B4 (Sigma-Aldrich) to prepare a 1 mg/ml stock solution. A small amount of the solution was aliquoted and stored in a deep freezer ultra-low freezer (TSX Series, Thermo Scientific^{™}).

### Experimental Example 2-3: Preparation of tumor growth factor β1 (TGF-β1) stock solution

A 2 µg/ml stock solution was prepared by adding a sterile 4 mM HCl solution containing 0.1% bovine serum albumin (BSA, Sigma-Aldrich) to Recombinant Human TGF beta 1 Protein (R&D Systems). A small amount of the solution was aliquoted and stored in a deep freezer ultra-low freezer (TSX Series, Thermo Scientific^{™}).

### Experimental Example 2-4: Cell culture adaptation for triple co-culture

By gradually reducing the concentration of FBS in each complete medium of a human hepatocellular carcinoma cell line (HuH-7), an immortalized mouse Kupffer cell line (ImKC) and a human hepatic stellate cell line (LX-2), the cells were adapted in a CO₂ incubator (Thermo Scientific^{™}) under 37°C and 5% CO₂ conditions for 2 weeks, such that the concentration was finally 1%.

### Experimental Example 2-5: Cell suspension preparation for triple co-culture

The human hepatocellular carcinoma cell line (HuH-7), the immortalized mouse Kupffer cell line (ImKC) and the human hepatic stellate cell line (LX-2) cultured in the CO₂ incubator (Thermo Scientific^{™}) were taken out, and washed once with 1X DPBS (HyClone^{™}) after discarding all the cell culture medium. All DPBS was discarded, an accutase solution (Sigma-Aldrich) was added thereto, and the resulting mixture was left to stand in a CO₂ incubator (Thermo Scientific^{™}) under 37°C and 5% CO₂ conditions for 3 minutes. Each of the cells and Dulbecco's Modified Eagle's Medium [+] 4.5 g/L glucose, L-glutamine and sodium pyruvate (DMEM, Corning) containing 1% P/S (HyClone^{™}) and 1% fetal bovine serum (FBS, Gibco), which is a co-culture medium, were mixed in a conical tube (SPL), and the resulting mixture was centrifuged at 1300 rpm for 3 minutes. After all the supernatant was removed from each conical tube (SPL), a new co-culture medium was added thereto, the cells were resuspended by tapping or pipetting, and the cell viability and cell number of each cell line were measured using an automated cell counter (Logos Biosystems).

### Experimental Example 2-6: Cell seeding for direct/indirect co-culture

### <Direct co-culture: Hepatocytes: HSCs>

Considering a 144-hour cell culture experiment with cell suspensions of the prepared human hepatocellular carcinoma cell line (HuH-7) and human hepatic stellate cell line (LX-2), the cells were seeded as shown in the following Table 1 such that the final cell confluency became 80 to 90% after 144 hours of cell seeding. A human hepatocellular carcinoma cell line (HuH-7) and a human hepatic stellate cell line (LX-2) were each put into a new conical tube (SPL), such that the cell number ratio of HuH-7 to LX-2 became 5 : 1, the cells were well mixed by tapping or pipetting, and then seeded in a cell culture dish, and the cells were fixed in a CO₂ incubator (Thermo Scientific^{™}) under 37°C and 5% CO₂ conditions for 1 hour (FIG. 2).

**[Table 1]**

| **Checkpoint (h)** | **Human Hepatocellular Carcinoma Cell Line** (cells/cm²) | **Human Hepatic Stellate Cell Line** (cells/cm²) | **Total Cell Number** (cells/cm²) |
|---|---|---|---|
| | **HuH**-7 | **LX-2** | |
| 144 | 12630 | 3160 | 15790 |

### <Triple co-culture: Direct co-culture + Indirect co-culture (Kupffer cells)>

After the cells were fixed for 1 hour for direct co-culture, a 0.4 µm pore size cell culture insert (Corning^{™}) was positioned on top of a direct co-culture dish for indirect co-culture of the corresponding cells with an immortalized mouse Kupffer cell line (ImKC). The cell suspensions of the immortalized mouse Kupffer cell line (ImKC) were added to the positioned cell culture insert (Corning^{™}) such that the number of cells per area was 15,000 cells/cm², and the cells were cultured in a CO₂ incubator (Thermo Scientific^{™}) under 37°C and 5% CO₂ conditions for 24 hours (FIG. 1).

### <Control: Mono-Culture>

Considering a 144-hour cell culture and an experiment with the suspensions of the human hepatocellular carcinoma cell line (HuH-7), immortalized mouse Kupffer cell line (ImKC) and human hepatic stellate cell line (LX-2) prepared as mono-culture controls for triple co-culture, the cell numbers were adjusted differently as shown in the following Table 2 such that the final cell confluency became 80 to 90% after 144 hours of cell seeding. Each cell suspension was added to a new conical tube (SPL), the cells were well mixed by tapping or pipetting, and then the resulting mixture was seeded in a cell culture dish, and cultured in a CO₂ incubator (Thermo Scientific^{™}) under 37°C and 5% CO₂ for 24 hours.

**[Table 2]**

| **Checkpoint (h)** | **Human Hepatocellular Carcinoma Cell Line** (cells/cm²) | **Human Hepatic Stellate Cell Line** (cells/cm²) | **Immortalized Mouse Kupffer Cell Line** (cells/cm²) |
|---|---|---|---|
| | **HuH**-7 | **LX-2** | **ImKC** |
| 144 | 16,000 | 6,300 | 40,000 |

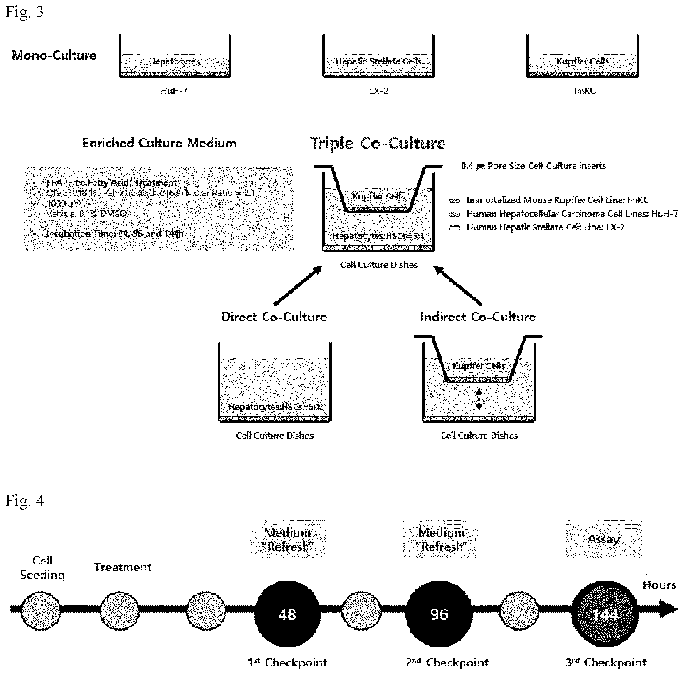

FIG. 3 is a schematic view illustrating the difference between mono-culture and triple co-culture methods.

### Experimental Example 2-7: Induction of in vitro hepatic steatosis by free fatty acid (FFA) treatment

### <FFA (oleic/palmitic acid) treatment>

The cell culture dishes for each experimental group shown in the following Table 3, which was cultured for 24 hours in a CO₂ incubator (Thermo Scientific^{™}) under 37°C and 5% CO₂ conditions at 24 hours before FFA treatment, were taken out, the cell culture medium was carefully discarded, and a co-culture medium including 1,000 µM FFA (oleic : palmitic molar ratio 2 : 1) was added thereto. After FFA treatment, the cell culture dishes were incubated in a CO₂ incubator (Thermo Scientific^{™}) under 37°C and 5% CO₂ conditions for 144 hours. The co-culture medium including FFA was replaced at 48 and 96 hours after FFA treatment (FIG. 4).

**[Table 3]**

| **Check point (h)** | **Condit ion** | **Human Hepatocellular Carcinoma Cell Line** | **Human Hepatic Stellate Cell Line** | **Immort alized Mouse Kupffer Cell Line** | **Direct Co-Culture (Hepatocytes** : **HSCs** = **5 : 1)** | **Triple Co-Culture (Direct/Indirect Co-Culture)** |
|---|---|---|---|---|---|---|
| | | **HuH-7** | **LX-2** | **ImKC** | **HuH-7:LX-2** | **HuH-7:LX-2:ImKC** |
| 144 | **Contr ol** | Vehicle | Vehicle | Vehicle | Vehicle | Vehicle |
| | **FFA (µM)** | 1,000 | 1,000 | 1,000 | 1,000 | 1,000 |

### Experimental Example 2-8: Induction of in vitro hepatic inflammation by FFA + LPS treatment

The cell culture dishes for each experimental group shown in Table 3, which was cultured in a CO₂ incubator (Thermo Scientific^{™}) under 37°C and 5% CO₂ conditions at 72 and 96 hours before LPS treatment, were taken out, the cell culture medium was carefully discarded, and a culture medium including 0.5 ng/ml LPS was added to 1,000 µM FFA (oleic : palmitic molar ratio 2 : 1). After LPS treatment, the cell culture dishes were incubated in a CO₂ incubator (Thermo Scientific^{™}) under 37°C and 5% CO₂ conditions for 48 and 72 hours.

### Experimental Example 2-9: Induction of in vitro hepatic fibrosis by FFA + TGF-β1 treatment

The cell culture dishes for each experimental group shown in Table 3, which was cultured in a CO₂ incubator (Thermo Scientific^{™}) under 37°C and 5% CO₂ conditions at 72 hours before TGF-β1 treatment, were taken out, the cell culture medium was carefully discarded, and a culture medium including 2 and 4 ng/ml TGF-β1 was added to 1,000 µM FFA (oleic : palmitic molar ratio 2 : 1). After TGF-β1 treatment, the cell culture dishes were incubated in a CO₂ incubator (Thermo Scientific^{™}) under 37°C and 5% CO₂ conditions for 72 hours.

### Experimental Example 3: Measurement of changes in various markers in triple co-culture system

### Experimental Example 3-1: Oil Red O Staining: Measurement of absorbance: Measurement of intracellular lipid content

### <Cell Counting Kit-8 Assay: Normalization>

For the normalization of Oil Red O Staining through the measurement of cell cytotoxicity in the FFA treatment group and each experimental group, cytotoxicity was confirmed by measuring absorbance at 450 num using a multimode microplate reader (Thermo Scientific^{™}) from an amount of tetrazolium salt reduced by intracellular dehydrogenase and converted into formazan using a Cell Counting Kit-8 (CCK-8, Dojindo Molecular Technologies). The cell culture dishes of each experimental group shown in Table 3, which was cultured in a CO₂ incubator (Thermo Scientific^{™}) under 37°C and 5% CO₂ conditions for 144 hours after FFA treatment, were taken out, and a Cell Counting Kit-8 (CCK-8, Dojindo Molecular Technologies) solution was each added in an amount of 1/10 of the cell culture medium. The resulting mixture was allowed to react in a CO₂ incubator (Thermo Scientific^{™}) for 2 hours. After treatment for 2 hours, the cell culture dishes of each experimental group were taken out, 100 µl of the culture medium was transferred to a 96-well plate, and then absorbance was measured at 450 nm using a multimode microplate reader (Thermo Scientific^{™}).

### <Oil Red O Staining>

In order to measure changes in the content of an intracellular lipid content by FFA treatment, an Oil Red O staining kit (Abcam) was used, an intracellular lipid content was stained using an Oil Red O solution included in the kit, and then absorbance was measured using a multimode microplate reader (Varioskan^{™} LUX, Thermo Scientific^{™}). After Cell Counting Kit-8 assay was completed, all the cell culture medium was discarded, and the cells were washed once with 1X DPBS (HyClone^{™}). The cells were treated with a 10% formaldehyde solution (Sigma-Aldrich) for 1 hour for fixation, washed twice with distilled water, and treated with 60% isopropanol (Sigma-Aldrich) for 5 minutes. An Oil Red O solution (Abcam) was added thereto, and the cells were stained at room temperature for 1 hour while gently shaking the resulting mixture in an orbital shaker (DAIHAN, Korea). The stained cells were washed three times or more with distilled water, observed under a microscope, and then washed twice or more with 60% isopropanol (Sigma-Aldrich) in order to measure intracellular lipid content, and then 100% isopropanol (Sigma-Aldrich) was added thereto, and Oil Red O stained with intracellular lipid was eluted for 30 minutes while gently shaking the resulting mixture in an orbital shaker (DAIHAN). To analyze the amount of eluted Oil Red O, absorbance was measured at 500 nm in comparison with 100% isopropanol (Sigma-Aldrich) which is a blank using a multimode microplate reader (Thermo Scientific^{™}).

### Experimental Example 3-2: Sirius Red Staining: Measurement of intracellular collagen

### <Cell Counting Kit-8 Assay: Normalization>

For the normalization of Sirius Red Staining through the measurement of cell cytotoxicity for FFA treatment group and each experimental group, cytotoxicity was confirmed by measuring absorbance at 450 num using a multimode microplate reader (Thermo Scientific^{™}) from an amount of tetrazolium salt reduced by intracellular dehydrogenase and converted into formazan using a Cell Counting Kit-8 (CCK-8, Dojindo Molecular Technologies). The cell culture dishes of each experimental group shown in Table 3, which was cultured in a CO₂ incubator (Thermo Scientific^{™}) under 37°C and 5% CO₂ conditions for 144 hours after FFA treatment, were taken out, and a Cell Counting Kit-8 (CCK-8, Dojindo Molecular Technologies) solution was each added in an amount of 1/10 of the cell culture medium. The resulting mixture was allowed to react in a CO₂ incubator (Thermo Scientific^{™}) for 2 hours. After treatment for 2 hours, the cell culture dishes of each experimental group were taken out, 100 µl of the culture medium was transferred to a 96-well plate, and then absorbance was measured at 450 nm using a multimode microplate reader (Thermo Scientific^{™}).

### <Sirius Red Staining>

In order to measure changes in the content of intracellular collagen by FFA treatment, a Picro-Sirius Red solution (Abcam) was used, and the absorbance of intracellular collagen stained with Picro-Sirius Red Dye was measured using a multimode microplate reader (Thermo Scientific^{™}). After Cell Counting Kit-8 assay was completed, all the cell culture medium was discarded, and the cells were washed once with 1X DPBS (HyClone^{™}). The cells were treated with Bouin's Solution (MEDILAB) for 1 hour for fixation, then washed by being immersed in running tap water for 20 minutes, and dried in the air. A Picro-Sirius Red solution (Abcam) was added to the dried cells, and the cells were stained at room temperature for 2 hours while gently shaking the resulting mixture in an orbital shaker (DAIHAN). The Picro-Sirius Red solution (Abcam) was removed from the cells, and a non-bound Picro-Sirius Red dye was removed by washing the residue with 0.01 N hydrochloric acid (Sigma-Aldrich). A 0.1 N sodium hydroxide solution (Sigma-Aldrich) was added thereto, and Picro-Sirius Red stained with intracellular collagen was eluted for 30 minutes while gently shaking the resulting mixture in an orbital shaker (DAIHAN). To analyze the amount of eluted Picro-Sirius Red, absorbance was measured at 550 nm in comparison with a 0.1 N sodium hydroxide solution which is a blank using a multimode microplate reader (Thermo Scientific^{™}).

### Experimental Example 3-3: qRT-PCR: Confirmation of mRNA expression of inflammation- and fibrosis-related molecules

### <Total RNA Extraction>

Total RNA was extracted from the FFA-treated experimental groups using a TRIzol^{™} reagent (Invitrogen). After a cell culture dish was taken out from the CO₂ incubator (Thermo Scientific^{™}), the cells were allowed to react with a TRIzol^{™} reagent (Invitrogen) for 5 minutes by adding the reagent thereto after discarding all the cell culture medium. The cells were resuspended by pipetting and transferred to a 1.5 mL microcentrifuge tube (Axygen). Chloroform (Sigma-Aldrich) was added to the mixture of TRIzol^{™} reagent, and the resulting mixture was vortexed, and then allowed to react at room temperature for 5 minutes. The mixture was centrifuged (12,000 xg, 15 minutes, 4°C) using a micro centrifuge (Labogene), the supernatant including total RNA was transferred to a new 1.5 mL microcentrifuge tube (Axygen), and then isopropanol (Sigma - Aldrich) was added thereto, the tube was inverted 2 to 3 times, and the cells were allowed to react at room temperature for 10 minutes. The tube including the mixture was centrifuged (12,000 xg, 15 minutes, 4°C) using a micro centrifuge (Labogene), the supernatant was removed, and the remaining total RNA pellet was secured. 75% ethanol (Sigma-Aldrich) was added to the total RNA pellet, and the total RNA pellet was mixed well by inverting the tube 2 to 3 times. The mixture was centrifuged (13,000 rpm, 5 minutes, 4°C) using a micro centrifuge (Labogene), the supernatant was removed, and the remaining total RNA pellet was dried at room temperature. The dried total RNA pellet was dissolved in nuclease-free water (Invitrogen), and absorbance was measured at 260 nm and 280 nm using a NanoDrop^{™} 2000 spectrophotometer (Thermo Scientific^{™}) to measure RNA purity (260/280 ratio) and concentration (ng/µL). When the 260/280 ratio was 1.9 or less as the exclusion criteria, it was determined that the RNA purity was low, and thus, the use in the experiment was excluded.

### <Complementary DNA (cDNA) Synthesis>

Complementary DNA (cDNA) by reverse transcription was synthesized from the extracted total RNA using amfiRivert cDNA Synthesis Platinum Master Mix (GenDEPOT, USA). After 2X RT Master Mix and Enzyme Mix were added to a 0.2 mL PCR tube (Axygen, USA), a total RNA sample was added thereto, the volume was adjusted using nuclease-free water (Invitrogen), and then the sample was mixed by pipetting. A PCR tube (Axygen) including the mixture was spun down using a micro centrifuge (Labogene), placed in ice, and stored. The PCR tube (Axygen) was transferred to a thermal cycler (MiniAmp^{™} Plus, Applied Biosystems), and cDNA was synthesized under the following conditions: Step 1 (25°C, 5 minutes), Step 2 (55°C, 60 minutes), Step 3 (70°C, 15 minutes), and Step 4 (4°C, Hold). The synthesized cDNA was stored in a deep freezer ultra-low freezer (TSX Series, Thermo Scientific^{™}).

### <qRT-PCR>

qRT-PCR was performed on the synthesized cDNA using Power SYBR^{™} Green PCR Master Mix (Applied Biosystems) and Real-Time PCR System (QuantStudio^{™} 3, Applied Biosystems). 2X Power SYBR^{®} Green PCR Master Mix (Applied Biosystems), cDNA, and forward/reverse primers were added to a 0.2 mL PCR tube (Axygen), the volume was adjusted using distilled water, and then the sample was mixed by pipetting. Information on qRT-PCR target molecules and primers used is shown in the following Table 4. A PCR tube (Axygen) including the mixture was spun down using a micro centrifuge (Labogene), and the mixture was transferred to each well of a 96-well reaction plate (MicroAmp^{®} Optical, Applied Biosystems). The 96-well reaction plate (MicroAmp^{®} Optical, Applied Biosystems) to which the mixture was transferred was sealed by attaching an adhesive film (MicroAmp^{®} Optical, Applied Biosystems) to the upper surface of the 96-well reaction plate, and centrifuged (3,000 rpm, 3 minutes) using a micro centrifuge (Labogene). The 96-well reaction plate containing the mixture was transferred to the Real-Time PCR System (Applied Biosystems), and thermal-cycling reaction was performed under the following conditions: enzyme activation (95°C, 10 minutes), PCR [40 cycles: denature (95°C, 5 seconds), anneal (60°C, 25 seconds), and extend (72°C, 30 seconds)]. A primer melting temperature (Tm) value for each target molecule was used by performing a thermal cycler (Applied Biosystems) before performing qRT-PCR to establish conditions. For result analysis, calculation was performed according to the manufacturer's manuals of Real-Time PCR System.

**[Table 4]**

| **1. Human (Huh-7, LX-2)** | | | | | |
|---|---|---|---|---|---|
| **Gene Name** | **[Accession Number** | **[Forward (5'-3')** | **[Reverse (5'-3')** | | |
| **Adenosine Receptors** | | | | | |
| ADORA3 (Adenosine A3 receptor) | NM_000677.4 | TGTCATGGCTGCCTT TATCT | CGATAGGGTTCA TCATGGAG | | |

| **Farnesoid X Receptor** | | | | | |
|---|---|---|---|---|---|
| NR1H4 (nuclear receptor subfamily 1, group H, member 4) | NM_001206977.2 | ACAGTGAAGGTCGT GACTTG | AGAAGAGTCTGT TGATCTGGG | | |

| **Thyroid Hormone Receptor** | | | | | |
|---|---|---|---|---|---|
| Thyroid Hormone Receptor Beta (THRB) | NM _ 000461.5 | AAGGTGGAAAGGTT GACTTGG | TCTTCACATGGC AGCTCAC | | |

| **Pro-Inflammatory Cytokines** | | | | | |
|---|---|---|---|---|---|
| TNF (Tumor Necrosis Factor α) | NM_000594.4 | TCCTCAGCCTCTTCT CCTTC | AGGGCTGATTAG AGAGAGGTC | | |
| CCL2 (C-C motif chemokine ligand 2) | NM_002982.4 | AGCCAGATGCAATC AATGCC | TTCTTTGGGACA CTTGCTGC | | |
| IL1B (Interleukin 1B) | NM_000576.3 | AGCAACAAGTGGTG TTCTCC | GGGATCTACACT CTCCAGC | | |
| IL6 (Interleukin 6) | NM_000600.5 | ACCTGAACCTTCCAA AGATGG | CTGGCTTGTTCCT CACTACTC | | |
| CXCL8 (Chemokine (C-X-C motif) ligand 8),Interleukin 8 | NM_000584.4 | CCTTCCTGATTTCTG CAGCTC | GGTGGAAAGGTT TGGAGTATGTC | | |

| **Fibrosis Markers** | | | | | |
|---|---|---|---|---|---|
| ACTA2 (α-Smooth Muscle Actin, α-SMA) | NM_001141945.2 | TGAGCGTGGCTATTC CTTC | CAGGCAACTCGT AACTCTTC | | |
| COL1A1 (Collagen, type I, α1) | NM_000088.4 | AGACGAAGACATCC CACCAA | ATCGCACAACAC CTTGCC | | |
| COL4A1 (Collagen, Type IV,α1) | NM_001845.6 | AAGGGTGATACTGG AGAACC | TTTCCAGGGTAG CCAGATG | | |
| TGFB1 (Transforming Growth Factor β1) | NM_000660.7 | ATTCCTGGCGATACC TCAGC | ATTTCCCCTCCA CGGCTCA | | |
| TIMP1 (Metallopeptidase Inhibitor 1) | NM_003254.3 | GATGGACTCTTGCAC ATCACT | GATAAACAGGGA AACACTGTGC | | |
| TIMP2 (Metallopeptidase Inhibitor 2) | NM_003255.5 | ATCTCATTGCAGGAA AGGCC | GTACCTGTGGTT CAGGCTCT | | |
| Lysyl Oxidase (LOX) | NM_002317.7 | GAGTTTAGCCACTAT GACCTGC | CTCAATCCCTGT GTGTGTGC | | |

| **House Keeping Genes: Internal Controls** | | | | | |
|---|---|---|---|---|---|
| HPRT | NM_000194.3 | AAGATGGTCAAGGT | AGTCAAGGGCAT | | |
| (Hypoxanthine PhosphoribosylTr ansferase 1, HPRT1) | | | CGCAAG | | ATCCTACAAC |

| **2. Mouse (Immortalized Kupffer Cell)** | | | | | |
|---|---|---|---|---|---|
| **Adenosine Receptors** | | | | | |
| ADORA3 (Adenosine A3 receptor) | | NM_009631.4 | | CACCCAT GCTTCCA TCATGT | TCTTCTTTGAGTG GTAACCGT |

| **Pro-Inflammatory Cytokines** | | | | | |
|---|---|---|---|---|---|
| CCL2 (C-C motif chemokine ligand 2) | | NM_011333.3 | | GGTCCCT GTCATGC TTCTG | TCCTCTTGTAGCT CTCCAGC |
| IL1B (Interleukin 1 beta | | NM_008361.4 | | AAAGCT CTCCACC TCAATG G | TCGTTGCTTGGTT CTCCTTG |
| IL6 (Interleukin 6) | | NM_031168.2 | | TGATGG ATGCTAC CAAACT GG | TCTCTCTGAAGG ACTCTGGC |
| TNF (Tumor Necrosis Factor α) | | NM_013693.3 | | GTCTCAG CCTCTTC TCATTCC | GGGAACTTCTCA TCCCTTTGG |

| **House Keeping Genes: Internal Controls** | | | | | |
|---|---|---|---|---|---|
| HPRT (Hypoxanthine PhosphoribosylTransferase 1, HPRT1) | | NM_013556.2 | | ATGGAC TGATTAT GGACAG GAC | CAGCAGGTCAGC AAAGAAC |

### Experimental Example 3-4: Western Blot Analysis: Confirmation of changes in A3AR expression

### <Whole Cellular Protein Extraction>

To extract whole cellular proteins from FFA treatment and experimental groups, cOmplete^{™} and EDTA-free protease inhibitor cocktail tablets (Roche) were added to 1X RIPA lysis buffer (Merck Millipore), dissolved and homogenized, and then the whole cellular proteins were extracted. A cell culture dish was taken out from the CO₂ incubator (Thermo Scientific^{™}) to discard all the cell culture medium, and 1X DPBS (HyClone^{™}) was added thereto. Cells were collected using a cell scraper and mixed with 1X DPBS (HyClone^{™}) in a conical tube (SPL), and the resulting mixture was centrifuged at 1,500 rpm for 4 minutes. DPBS was partially removed, and the cells were resuspended by tapping or pipetting, transferred to a 1.5 mL microcentrifuge tube (Axygen), and centrifuged at 1,500 rpm for 4 minutes using a micro centrifuge (Labogene). All DPBS was discarded, 1X RIPA lysis buffer was added thereto, pipetting was performed, and then the cells were placed in ice and homogenized while being allowed to react for 30 minutes. The homogenized sample was centrifuged (13,000 rpm, 20 minutes, 4°C) using a micro centrifuge (Labogene), and the supernatant was isolated. The amount of whole cellular protein included in the isolated supernatant was quantified by measuring absorbance at 595 nm with a multimode microplate reader (Thermo Scientific^{™}) using a Bio-Rad protein assay kit (Bio-Rad).

### <Sodium Dodecyl Sulphate-Polyacrylamide Gel Electrophoresis (SDS-PAGE)>

Proteins isolated by centrifugation and 4x Laemmli Sample Buffer (Bio-Rad, USA) were mixed at a ratio of 3 : 1, and then boiled at 95°C for 10 minutes to denature the proteins, and then cooled on ice to prepare a Western blot sample. For SDS-PAGE, Mini-PROTEAN TGX Gel (Bio-Rad) was placed on a Mini-Protein II Dual-Slab apparatus (Bio-Rad), then the apparatus was filled with a running buffer (Tris/Glycine/SDS buffer, Bio-Rad), a protein size marker (Bio-Rad) and each sample were aliquoted into each well of the Mini-PROTEAN TGX Gel, and electrophoresis was performed at 150 volt until each sample reached the bottom to isolate proteins according to the molecular weights thereof.

### <Western Blot Transfer>

A nitrocellulose blotting membrane (Amersham^{™}) soaked with a transfer buffer and a mini trans-blot filter paper (Bio-Rad) soaked with the transfer buffer were layered in order and mounted on Mini Trans-Bolt Cell (Bio-Rad), followed by transfer at 200 mA for 60 minutes, and when the protein transfer was completed with the nitrocellulose membrane (Bio-Rad), the NC membrane (Amersham^{™}) was blocked with a 5% (v/v) skim milk solution [Tris buffered saline (TBS) buffer (Bio-Rad), 0.1% Tween^{™} 20 (Thermo Scientific^{™}), and 5% Difco^{™} skim milk (BD Bioscience)] on an orbital shaker for 60 minutes.

### <Antibody Incubation>

The primary antibodies used and treatment conditions for Western Blot analysis are shown in the following Table 5, and GAPDH (Cell Signaling Technology) was used as a primary antibody for the Western blot loading control. After the primary antibodies were each appropriately diluted in a 5% (w/v) skim milk solution, protein transfer was performed while shaking the resulting mixture using an orbital shaker (DAIHAN) at 4°C for 16 hours, followed by treatment with an NC membrane (Amersham^{™}) in which blocking was completed. The primary antibody solution was collected, the PVDF membrane (Bio-Rad) was washed with a TBS-T solution [TBS buffer (Bio-Rad), 0.1% Tween^{™} 20 (Thermo Scientific^{™})], and then secondary antibodies were diluted in a 5% (w/v) skim milk solution and treated at room temperature for 60 minutes under shaking using an orbital shaker (DAIHAN). The secondary antibody solution was collected, and the NC Membrane (Amersham^{™}) was washed with a TBS-T solution under shaking.

The following Table 5 summarizes information on primary & secondary antibodies for Western Blot analysis.

**[Table 5]**

| **Antibody** | **Dilution Ratio** | **Catalog No** | **Company** |
|---|---|---|---|
| **Adenosine Receptors** | | | |
| Adenosine A3 Receptor | 1:500 | bs-1225R | Bioss Antibodies |

| **Loading Control** | | | |
|---|---|---|---|
| GAPDH | 1:1,000 | sc-32233 | SantaCruz Biotechnology |

| **Secondary Antibody** | | | |
|---|---|---|---|
| Goat Anti-rabbit lgG, HRP conjugated | 1:5,000 | AbC-5003 | Abclon |
| Goat Anti-mouse lgG, HRP conjugated | 1:5,000 | AbC-5001 | Abclon |

### <Protein Detection and Analysis>

The secondary antibody was treated, the washed NC membrane (Amersham^{™}) was added to SuperSignal^{™} West Femto Maximum Sensitivity Substrate (Thermo Scientific^{™}), color was developed for each appropriate time, and then scanning was performed using ImageQuant^{™} LAS 500 (GE Healthcare).

### Experimental Example 4: Measurement of changes in various markers of related molecules by treatment with FM101 (A3AR ligand) and control material

### Experimental Example 4-1: Compound stock preparation of FM101 and control materials

After an FM101 material represented by the following Chemical Formula A and the control materials to be used in the test were dissolved in dimethyl sulfoxide (DMSO) at 10 mM, the resulting solutions were aliquoted and stored in a deep freezer ultra-low freezer (TSX Series, Thermo Scientific^{™}) (Table 6). It was confirmed that the FM101 material modulates an A₃ adenosine receptor as an A₃AR ligand in *in vivo* experiments (EXCLI J. 2020 Feb 12;19:187-200.).

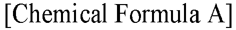

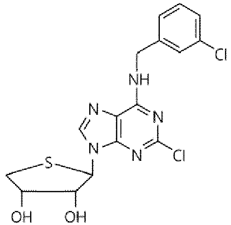

**[Table 6]**

| **Compound** | **Description** | **Molecular Weight (g/mol)** | **Stock Concentration (mM)** |
|---|---|---|---|
| FM101 | A3 Adenosine Receptor Ligand | 412.29 | 10 |
| Obeticholic Acid (INT-747, Ocaliva, OCA) | Farnesoid-X-Activated Receptor (FXR) Agonist | 420.6 | 10 |
| Namodenoson (CF-102) | A3 Adenosine Receptor Agonist | 544.7 | 10 |
| Resmetirom (MGL-3196; | Thyroid Hormone Receptor (THR-β) Agonist | 435.221 | 10 |

### Experimental Example 4-2: Oil Red O Staining: Measurement of absorbance: Measurement of changes in intracellular lipid Droplets by treatment with FM101 and control material

### <Cell Counting Kit-8 Assay: Normalization>

The absorbance of each cell culture treated with FM101 and the control material was measured in the same manner as in Experimental Example 3-1.

### <Oil Red O Staining>

The absorbance of each cell culture treated with FM101 and the control material was measured in the same manner as in Experimental Example 3-1.

### Experimental Example 4-3: Sirius Red Staining: Measurement of intracellular collagen: Measurement of changes in intracellular collagen by treatment with FM101 and control material

### <Cell Counting Kit-8 Assay: Normalization>

The absorbance of each cell culture treated with FM101 and the control material was measured in the same manner as in Experimental Example 3-2.

### <Sirius Red Staining>

The absorbance of each cell culture treated with FM101 and the control material was measured in the same manner as in Experimental Example 3-2.

### Experimental Example 4-4: qRT-PCR: Confirmation of changes in mRNA expression of inflammation- and fibrosis-related molecules by treatment with FM101 and control material

### <Total RNA Extraction>

RNA purity and concentration were measured by measuring the absorbance of each cell culture treated with FM101 and the control material in the same manner as in Experimental Example 3-3.

### <Complementary DNA (cDNA) Synthesis>

cDNA was synthesized for each cell culture treated with FM101 and the control material in the same manner as in Experimental Example 3-3.

### <qRT-PCR>

qRT-PCR was performed on each cell culture treated with FM101 and the control material in the same manner as in Experimental Example 3-3.

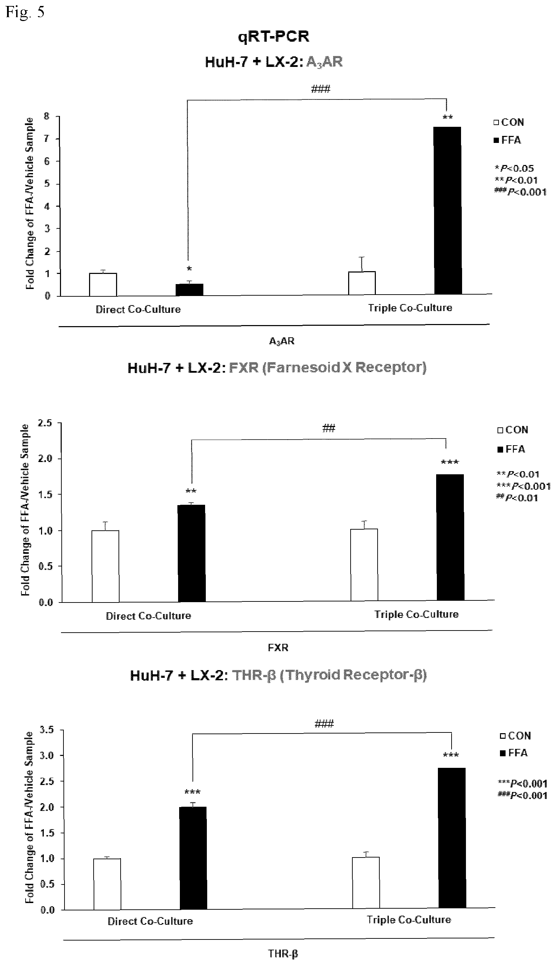

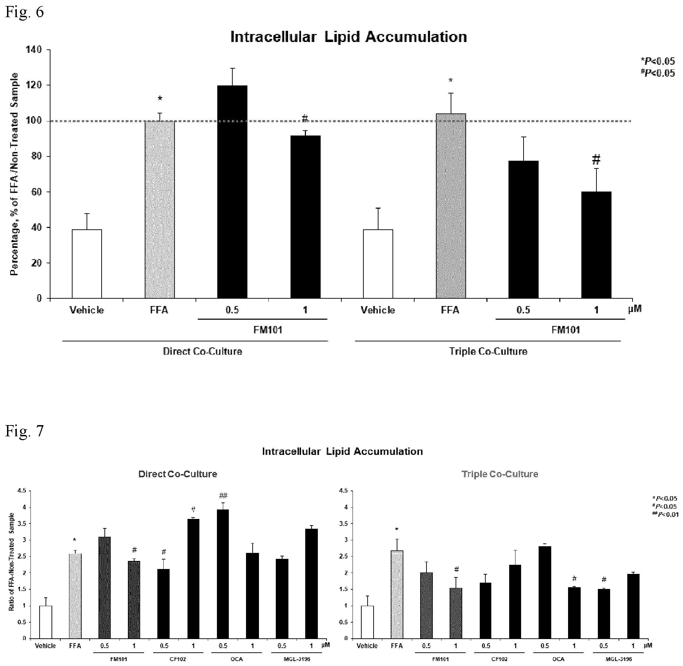

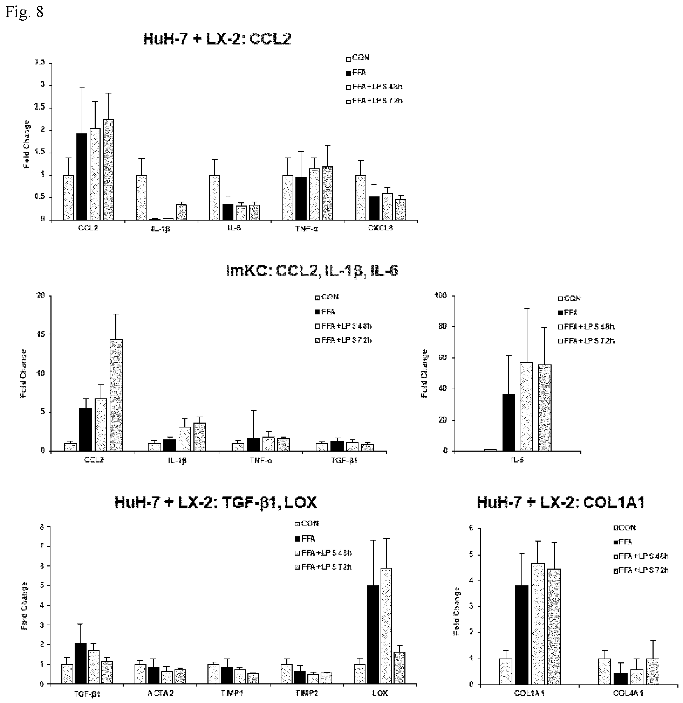

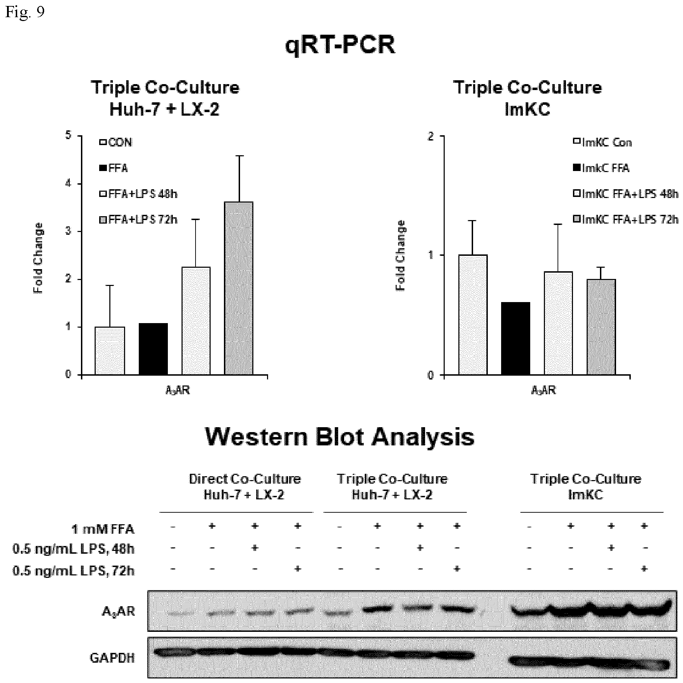

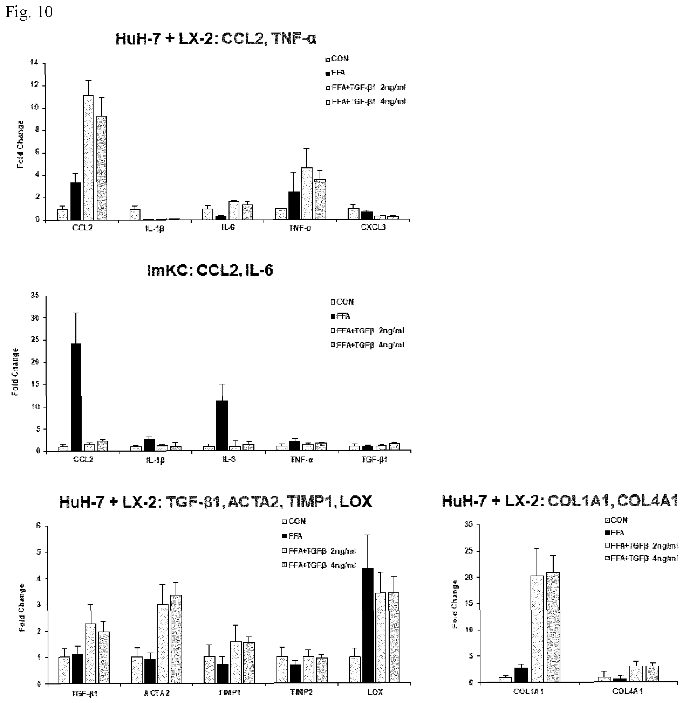

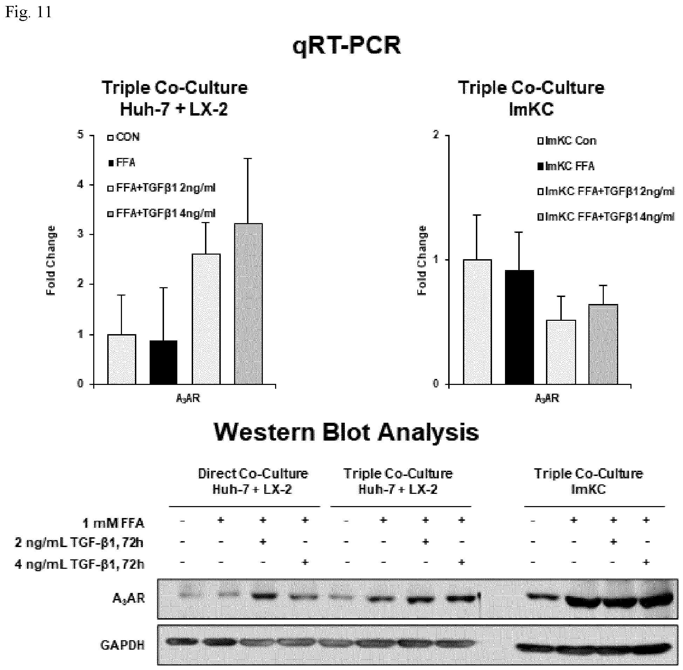

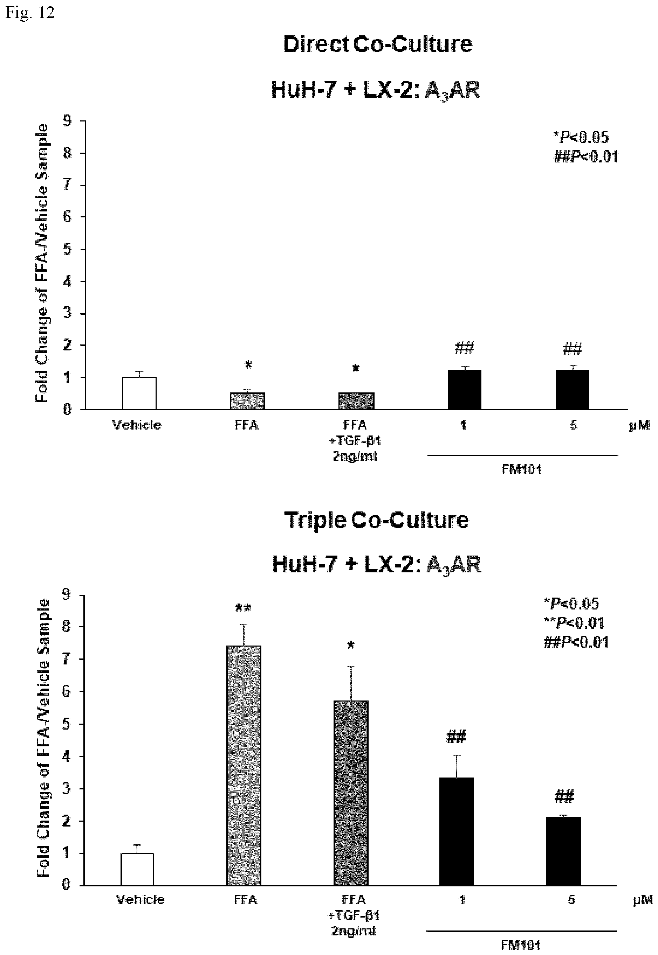

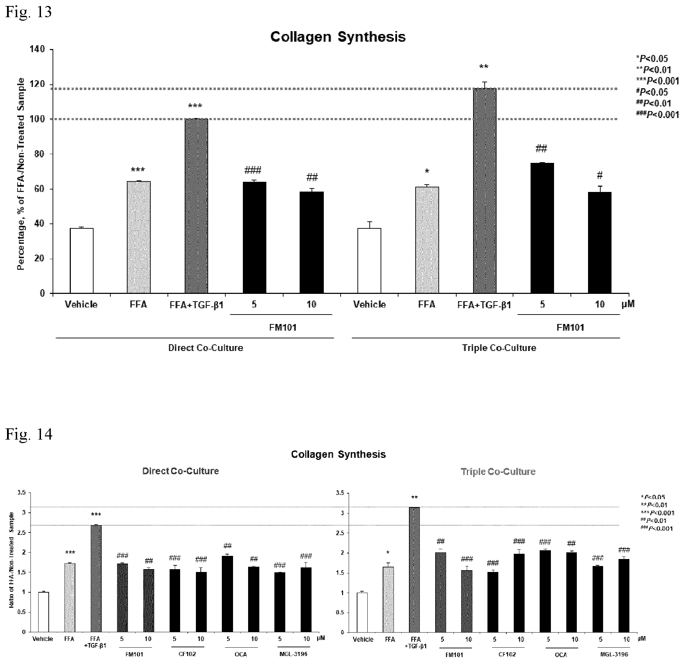

FIGS. 5 to 14 are graphs showing the results of Experimental Examples 3 and 4. FIGS. 5 to 7 illustrate the expression levels of markers such as A₃AR, FXR, THR-β, and lipid for cells in which hepatic steatosis was induced in Experimental Examples 2-7, FIGS. 8 and 9 illustrate the expression levels of markers such as CCL2, IL-1β, IL-6, and A₃AR for cells in which hepatitis was induced Experimental Example 2-8, and FIGS. 10 to 14 illustrate the expression levels of markers such as CCL2, TNF-a, A₃AR, and collagen for cells in which hepatic fibrosis was induced in Experimental Example 2-9.

As illustrated in FIGS. 5 to 14, the cells to which the triple co-culture liver disease model of the present invention was applied showed changes in various markers, which are different from those of directly co-cultured cells, when hepatic steatosis, hepatic inflammation, or hepatic fibrosis was induced. Furthermore, when treated with drugs such as FM101 (A₃AR ligand), CF102 (A₃AR agonist), OCA (FXR agonist) and MGL-3196 (THR-β agonist), cells showed higher responsiveness and more concentration-dependent responsiveness than those of directly co-cultured cells. In particular, the triple co-culture liver disease model showed remarkably excellent responsiveness and accuracy in terms of changes in expression level of A₃AR when treated with FFA and the like and changes in expression level of A₃AR when treated with FM101, compared to directly co-cultured cells.

The *in vitro* liver disease model using triple co-culture according to the embodiments of the present invention and the preparation method thereof enable a rapid and highly accurate analysis by similarly implementing the *in vivo* environment compared to mono-culture, and simultaneously make it easier to collect and measure specimens than typical direct co-culture.

The effects according to the embodiments of the present invention are not limited to the contents exemplified above, and more various effects are included in the present specification.

As described above, although the present invention is mainly described with reference to the embodiments of the present invention, this is merely an example and does not limit the present invention, and it will be appreciated that a person with ordinary skill in the art to which the present invention pertains can make various modifications and applications which are not exemplified above within a range not departing from the essential characteristics of the embodiments of the present invention. For example, each constituent element specifically shown in the embodiments of the present invention can be modified and implemented. And differences related to these modifications and applications should be construed as being included in the scope of the present invention defined in the appended claims.

## Claims

1. A method of preparing an *in vitro* liver disease model using triple co-culture, the method comprising directly co-culturing hepatocytes and hepatic stellate cells, and simultaneously
indirectly co-culturing Kupffer cells by being separated from the hepatocytes and the hepatic stellate cells.

2. The method of claim 1, wherein the hepatocytes and the hepatic stellate cells are cultured so as to contact each other, and
the Kupffer cells are cultured so as not to contact the hepatocytes and the hepatic stellate cells,
provided that the hepatocytes, the hepatic stellate cells, and the Kupffer cells share the same medium.

3. The method of claim 1, wherein the hepatocytes are one or more of HepG2 cells and HuH-7 cells.

4. The method of claim 1, wherein the hepatic stellate cells are LX-2 cells.

5. The method of claim 1, wherein the Kupffer cells are immortalized Kupffer cells.

6. The method of claim 1, further comprising inducing steatosis, inflammation or fibrosis in the hepatocytes, the hepatic stellate cells, and/or the Kupffer cells.

7. The method of claim 6, wherein the inducing of the steatosis is treating the hepatocytes, the hepatic stellate cells, and/or the Kupffer cells with a free fatty acid (FFA),
the inducing of the inflammation is treating the hepatocytes, the hepatic stellate cells, and/or the Kupffer cells with a FFA and a lipopolysaccharide (LPS), and
the inducing of the fibrosis is treating the hepatocytes, the hepatic stellate cells, and/or the Kupffer cells with a FFA and TGF-β1.

8. The method of claim 6, further comprising treating the hepatocytes, the hepatic stellate cells, and/or the Kupffer cells with one or more agonists, partial agonists, antagonists, or partial antagonists selected from the group consisting of A₁AR, A_{2A}AR, A_{2B}AR, A₃AR, β-arrestin, FXR and THR-β.

9. The method of claim 6, further comprising measuring one or more markers selected from the group consisting of steatosis, inflammation and fibrosis for the hepatocytes, the hepatic stellate cells and/or the Kupffer cells.

10. The method of claim 9, wherein the marker is one or more selected from the group consisting of A₃AR, FXR, THR-β, CCL2, LOX, COL1A1, COL4A1, IL-6, IL-1β, TNF-α, ACTA2, TIMP1, TIMP2, TGF-β1, CXCL8, lipid, collagen, reactive oxygen species (ROS), α-SMA and fibronectin.

11. The method of claim 1, wherein after 144 hours from a start of culture, a cell number ratio of the hepatocytes to the hepatic stellate cells is 2 to 7 : 1.

12. The method of claim 1, wherein after 144 hours from a start of culture, a cell number ratio of the sum of the hepatocytes and the hepatic stellate cells to the Kupffer cells is 1 : 1 to 5.

13. The method of any one of claims 1 to 12, wherein the liver disease is non-alcoholic steatohepatitis (NASH) and/or non-alcoholic fatty liver disease (NAFLD).

14. An *in vitro* liver disease model using triple co-culture, the model comprising: a first incubator containing hepatocytes and hepatic stellate cells; and
a second incubator containing Kupffer cells, which are disposed while being separated from the hepatocytes and the hepatic stellate cells,
wherein the *in vitro* liver disease model has pores with a size smaller than the Kupffer cell, which are formed on the bottom surface of the second incubator, and
further comprises a medium which fills both the first incubator and the second incubator through the pores.

15. The *in vitro* liver disease model of claim 14, wherein the pores have an average size of 0.1 to 0.6 µm.

16. The *in vitro* liver disease model of claim 14, wherein the medium comprises one or more selected from the group consisting of fetal bovine serum (FBS), penicillin/streptomycin (P/S), glutamine, glucose, sodium pyruvate, P/S (HyClone^{™}), Dulbecco's Modified Eagle's Medium, EmbryoMax^{™} 2 mM L-glutamine, EmbryoMax^{™} ES cell qualified FBS and EmbryoMax^{™} DMEM high glucose.
